# EUROPEAN PATENT APPLICATION

(11) **EP 2 377 810 A1**
(43) Date of publication of application: **19.10.2011**
(21) Application number: 09832037.7
(22) Date of filing: 25.09.2009
(51) Int. Cl.: B82B 1/00

(54) **REGULAR HEXAHEDRAL OR OCTAHEDRAL FERRITE NANOPARTICLE, AND METHOD FOR PRODUCING SAME**

(30) Priority: 12.12.2008 KR 20080126846
(71) Applicant: Snu RDB Foundation, Seoul 151-742 (KR)
(72) Inventor: HYEON, Taeghwan, Seoul 135-270 (KR); KIM, Dokyoon, Seoul 151-744 (KR)
(74) Representative: Rystedt, Per Hampus
(86) International application number: PCT/KR2009/005478
(87) International publication number: WO 2010/067951

(57) **Abstract**

The present invention relates to cubic or octahedral ferrite nanoparticles and a method for preparing the same. In particular, the present invention is directed to a ferrite nanocube which is superparamagnetic or ferromagnetic, and a method for preparing a ferrite nanocube, comprising heating a mixture of a metal precursor, a surfactant and a solvent.

## Description

### Technical Field

The present invention relates to cubic or octahedral ferrite nanoparticles and a method for preparing the same. In particular, the present invention is directed to a ferrite nanocube which is superparamagnetic or ferromagnetic, and a method for preparing a ferrite nanocube, comprising heating a mixture of a metal precursor, a surfactant and a solvent.

### Background Art

Ferrite is iron or a solid solution including iron as its principal component, and has a body centered cubic crystal structure.

Magnetite (Fe₃O₄), one of the most typical ferrites, is a ferrimagnetic mineral and is one of iron oxides belonging to spinel group. Magnetite is the strongest magnetic material out of the naturally present minerals in the Earth, and has been used for various apparatuses, such as a compass. Conventionally, magnetites had been produced by the Massart method in which FeCl₂ and FeCl₃ are added into an aqueous NaOH solution and reacted. The magnetites produced by the Massart method have a disadvantage that the magnetite particles are not uniform.

Hou et al. reported the method for synthesizing wüstite (FeO) nanoparticles with spherical or truncated-octahedral shape by thermolysis of iron (II) acetylacetonate in a mixture solution of oleic acid and oleylamine (Yanglong Hou, Zhichuan Xu, and Shouheng Sun, Controlled Synthesis and Chemical Conversions of FeO Nanoparticles, Angew. Chem., 2007,119, 6445-6448).

The thus synthesized wüstite (FeO) nanoparticles are antiferromagnetic at room temperature and, thus, are weakly magnetic and chemically unstable.

Magnetite nanoparticles prepared by Sun et al. have spherical shapes and sizes of below 10 nm. Thus, they are superparamagnetic at room temperature and weakly magnetic. In addition, it is difficult to produce cubic arrays with spherical nanoparticles.

As mentioned above, novel technologies for ferrite nanoparticles, particularly magnetite nanopartilces, and methods for preparing thereof have been developed. However, ferrite nanoparticles which are cubic or octahedral and superparamagnetic or ferrimagnetic at room temperature, and methods for preparing thereof have not yet been disclosed in the art.

Since the direction of magnetic field of superparamagnetic nanoparticles easily changes with temperature, the directions of magnetic field of each nanoparticle cannot be kept in a certain direction. In contrast, since the energy required for changing the direction of magnetic field of ferrimagnetic nanoparticles is higher than the thermal energy at room temperature, the directions of magnetic field of each nanoparticle remain unchanged. It is expected that these two nanoparticles are to be useful for drug delivery via external magnetic field, hyperthermia, MRI constrasting agents, etc.

### Technical Problem

The primary object of the present invention is to provide a ferrite nanocube which is superparamagnetic or ferromagnetic.

Another object of the present invention is to provide a cubic array comprising ferrite nanocubes which are ferrimagnetic.

Yet another object of the present invention is to provide an octahedral or truncated-cubic ferrite nanoparticle which is ferrimagnetic.

Still another object of the present invention is to provide a method for preparing a ferrite nanocube, comprising heating a mixture of a metal precursor, a surfactant and a solvent.

### Technical Solution

The aforementioned primary object of the present invention can be achieved by providing a ferrite nanocube which is superparamagnetic or ferromagnetic.

The "nanocube" in the present invention refers to a nanometer-sized cubic particle.

The ferrite may be magnetite (Fe₃O₄), bimetallic ferrite or magnetite doped with a metal. In addition, the bimetallic ferrite may be CoFe₂O₄, MnFe₂O₄, ZnFe₂O₄ or BaFe₁₂O₁₉, and the magnetite doped with a metal may be magnetite doped with Co, Mn, Ni, Zn or Ba.

Preferably, the ferrite nanocube may have a size of 10 nm to 200 nm.

Another object of the present invention can be achieved by providing a cubic array comprising ferrite nanocubes which are ferrimagnetic.

The "cubic array" in the present invention refers to three-dimensionally laminated ferrite nanoparticles. These orderly array of nanoparticles facilitate access to each nanoparticle and, thus, may aid improvements of magnetic storage media, magnetic sensors, etc.

The ferrite constituting the cubic array of the present invention may be magnetite (Fe₃O₄), bimetallic ferrite or magnetite doped with a metal. In addition, the bimetallic ferrite may be CoFe₂O₄, MnFe₂O₄, ZnFe₂O₄ or BaFe₁₂O₁₉, and the magnetite doped with a metal may be magnetite doped with Co, Mn, Ni, Zn or Ba.

The yet another object of the present invention can be achieved by providing an octahedral or truncated-cubic ferrite nanoparticle which is ferrimagnetic. The octahedral nanoparticles, contrary to spherical nanoparticles which are symmetrical in all directions, show high magnetism in a certain direction and, thus, give more degree of freedom in respect of measuring magnetism of nanoparticles and utilizing thereof. Moreover, iron atoms present at the edges of octahedron have higher energy than those present at the surface of spheres and, therefore, may show higher reactivity.

The octahedral or truncated-cubic ferrite nanoparticle of the present invention may be magnetite (Fe₃O₄), bimetallic ferrite or magnetite doped with a metal. In addition, the bimetallic ferrite may be CoFe₂O₄, MnFe₂O₄, ZnFe₂O₄ or BaFe₁₂O₁₉, and the magnetite doped with a metal may be magnetite doped with Co, Mn, Ni, Zn or Ba.

Preferably, the octahedral or truncated-cubic ferrite nanoparticle may have a size of 10 nm to 200 nm.

The still another object of the present invention can be achieved by providing a method for preparing a ferrite nanocube, comprising heating a mixture of a metal precursor, a surfactant and a solvent.

When an iron precursor is used as the metal precursor, a ferrite nanocube may be prepared. Preferably, the iron precursor is one selected from the group consisting of iron (II) nitrate (Fe(NO₃)₂), iron (III) nitrate (Fe(NO₃)₃), iron (II) sulfate (FeSO₄), iron (III) sulfate (Fe₂(SO₄)₃), iron (II) acetylacetonate (Fe(acac)₂), iron (III) acetylacetonate (Fe(acac)₃), iron (II) trifluoroacetylacetonate (Fe(tfac)₂), iron (III) trifluoroacetylacetonate (Fe(tfac)₃), iron (II) acetate (Fe(ac)₂), iron (III) acetate (Fe(ac)₃), iron (II) chloride (FeCl₂), iron (III) chloride (FeCl₃), iron (II) bromide (FeBr₂), iron (III) bromide (FeBr₃), iron (II) iodide (FeI₂), iron (III) iodide (FeI₃), iron perchlorate (Fe(ClO₄)₃), iron sulfamate (Fe(NH₂SO₃)₂, iron (II) stearate ((CH₃(CH₂)₁₆COO)₂Fe), iron (III) stearate ((CH₃(CH₂)₁₆COO)₃Fe), iron (II) oleate ((CH₃(CH₂)₇CHCH(CH₂)₇COO)₂Fe), iron (III) oleate ((CH₃(CH₂)₇CHCH(CH₂)₇COO)₃Fe), iron (II) laurate ((CH₃(CH₂)₁₀COO)₂Fe), iron (III) laurate ((CH₃(CH₂)₁₀COO)₃Fe), iron pentacarbonyl (Fe(CO)₅), diiron nonacarbonyl (Fe₂(CO)₉) and disodium iron tetracarbonyl (Na₂[Fe(CO)₄]), or a mixture thereof.

In addition, when a mixture of an iron precursor and one selected from the group consisting of a cobalt precursor, a manganese precursor, a nickel precursor, a zinc precursor and a barium precursor is used, a bimetallic ferrite nanocube such as CoFe₂O₄, MnFe₂O₄, ZnFe₂O₄ or BaFe₁₂O₁₉, may be prepared, respectively.

The cobalt precursor may be selected from the group consisting of cobalt (II) nitrate (Co(NO₃)₂), cobalt (II) sulfate (CoSO₄), cobalt (II) acetylacetonate (Co(acac)₂), cobalt (II) trifluoroacetylacetonate (Co(tfac)₂), cobalt (II) acetate (Co(ac)₂), cobalt (II) chloride (CoCl₂), cobalt (II) bromide (CoBr₂), cobalt (II) iodide (CoI₂), cobalt sulfamate (Co(NH₂SO₃)₂, cobalt (II) stearate ((CH₃(CH₂)₁₆COO)₂Co), cobalt (II) oleate ((CH₃(CH₂)₇CHCH(CH₂)₇COO)₂Co), cobalt (II) laurate ((CH₃(CH₂)₁₀COO)₂Co) or dicobalt octacarbonyl (Co₂(CO)₈)).

The manganese precursor may be selected from the group consisting of manganese (II) nitrate (Mn(NO₃)₂), manganese (II) carbonate (MnCO₃), manganese (III) nitrate (Mn(NO₃)₃), manganese (II) sulfate (MnSO₄), manganese (III) sulfate (Mn₂(SO₄)₃), manganese (II) acetylacetonate (Mn(acac)₂), manganese (III) acetylacetonate (Mn(acac)₃), manganese (II) trifluoroacetylacetonate (Mn(tfac)₂), manganese (III) trifluoroacetylacetonate (Mn(tfac)₃), manganese (II) acetate (Mn(ac)₂), manganese (III) acetate (Mn(ac)₃), manganese (II) chloride (MnCl₂), manganese (II) bromide (MnBr₂), manganese (II) iodide (MnI₂), manganese perchlorate (Mn(ClO₄)₃), manganese sulfamate (Mn(NH₂SO₃)₂), manganese (II) stearate ((CH₃(CH₂)₁₆COO)₂Mn), manganese (III) stearate ((CH₃(CH₂)₁₆COO)₃Mn), manganese (II) oleate ((CH₃(CH₂)₇CHCH(CH₂)₇COO)₂Mn), manganese (III) oleate ((CH₃(CH₂)₇CHCH(CH₂)₇COO)₃Mn), manganese (II) laurate (CH₃(CH₂)₁₀COO)₂Mn), manganese (III) laurate (CH₃(CH₂)₁₀COO)₃Mn), dimanganese decacarbonyl (Mn₂(CO)₁₀) or manganese (II) methoxide (Mn(OMe)₂).

The nickel precursor may be selected from the group consisting of nickel (II) nitrate (Ni(NO₃)₂), nickel (II) sulfate (NiSO₄), nickel (II) acetylacetonate (Ni(acac)₂), nickel (II) trifluoroacetylacetonate (Ni(tfac)₂), nickel (II) acetate (Ni(ac)₂), nickel (II) chloride (NiCl₂), nickel (II) bromide (NiBr₂), nickel (II) iodide (NiI₂), nickel sulfamate (Ni(NH₂SO₃)₂, nickel (II) stearate ((CH₃(CH₂)₁₆COO)₂Ni), nickel (II) oleate ((CH₃(CH₂)₇CHCH(CH₂)₇COO)₂Ni), nickel (II) laurate ((CH₃(CH₂)₁₀COO)₂Ni) or nickel tetracarbonyl (Ni(CO)₄).

The zinc precursor may be selected from the group consisting of zinc (II) nitrate (Zn(NO₃)₂), zinc (II) sulfate (ZnSO₄), zinc (II) acetylacetonate (Zn(acac)₂), zinc (II) trifluoroacetylacetonate (Zn(tfac)₂), zinc (II) acetate (Zn(ac)₂), zinc (II) chloride (ZinCl₂), zinc (II) bromide (ZnBr₂), zinc (II) iodide (ZnI₂), zinc sulfamate (Zn(NH₂SO₃)₂ zinc (II) stearate ((CH₃(CH₂)₁₆COO)₂Zn), zinc (II) oleate ((CH₃(CH₂)₇CHCH(CH₂)₇COO)₂Zn), zinc (II) laurate ((CH₃(CH₂)₁₀COO)₂Zn) or zinc (II) tertiary-butoxide (Zn(*t*-butoxide)₂).

The barium precursor may be selected from the group consisting of barium (II) nitrate (Ba(NO₃)₂), barium (II) sulfate (BaSO₄), barium (II) acetylacetonate (Ba(acac)₂), barium (II) trifluoroacetylacetonate (Ba(tfac)₂), barium (II) acetate (Ba(ac)₂), barium (II) chloride (BaCl₂), barium (II) bromide (BaBr₂), barium (II) iodide (BaI₂), barium sulfamate (Ba(NH₂SO₃)₂, barium (II) stearate ((CH₃(CH₂)₁₆COO)₂Ba), barium (II) oleate ((CH₃(CH₂)₇CHCH(CH₂)₇COO)₂Ba), barium (II) laurate ((CH₃(CH₂)₁₀COO)₂Ba) or barium (II) isopropoxide (Ba(*i-*Pr)₂).

Preferably, the iron precursor used for preparing the ferrite nanocube of the present invention is one selected from the group consisting of iron (II) nitrate (Fe(NO₃)₂), iron (III) nitrate (Fe(NO₃)₃), iron (II) sulfate (FeSO₄), iron (III) sulfate (Fe₂(SO₄)₃, iron (II) acetylacetonate (Fe(acac)₂), iron (III) acetylacetonate (Fe(acac)₃), iron (II) trifluoroacetylacetonate (Fe(tfac)₂), iron (III) trifluoroacetylacetonate (Fe(tfac)₃), iron (II) acetate (Fe(ac)₂), iron (III) acetate (Fe(ac)₃), iron (II) chloride (FeCl₂), iron (III) chloride (FeCl₃), iron (II) bromide (FeBr₂), iron (III) bromide (FeBr₃), iron (II) iodide (FeI₂), iron (III) iodide (FeI₃), iron perchlorate (Fe(ClO₄)₃), iron sulfamate (Fe(NH₂SO₃)₂, iron (II) stearate ((CH₃(CH₂)₁₆COO)₂Fe), iron (III) stearate ((CH₃(CH₂)₁₆COO)₃Fe), iron (II) oleate ((CH₃(CH₂)₇CHCH(CH₂)₇COO)₂Fe), iron (III) oleate ((CH₃(CH₂)₇CHCH(CH₂)₇COO)₃Fe), iron (II) laurate ((CH₃(CH₂)₁₀COO)₂Fe), iron (III) laurate ((CH₃(CH₂)₁₀COO)₃Fe), iron pentacarbonyl (Fe(CO)₅), diiron nonacarbonyl (Fe₂(CO)₉) and disodium iron tetracarbonyl (Na₂[Fe(CO)₄]), or a mixture thereof.

Moreover, when the amount of the cobalt precursor, the manganese precursor, the nickel precursor, the zinc precursor or the barium precursor is used much smaller than that of the iron precursor, ferrite doped with cobalt, manganese, nickel, zinc or barium is obtained, respectively.

Preferably, the surfactant is selected from the group consisting of carboxylic acid, alkylamine, alkyl alcohol or alkylphosphine, or a mixture thereof.

Preferably, the carboxylic acid is selected from the group consisting of octanoic acid, decanoic acid, lauric acid, hexadecanoic acid, oleic acid, stearic acid, benzoic acid or biphenylcarboxylic acid, or a mixture thereof.

Preferably, the alkylamine is selected from the group consisting of octylamine, trioctylamine, decylamine, dodecylamine, tetradecylamine, hexadecylamine, oleylalmine, octadecylamine, tribenzylamine or triphenylamine, or a mixture thereof.

Preferably, the alkyl alcohol is selected from the group consisting of octylalcohol, decanol, hexadecanol, hexadecandiol, oleyl alcohol or phenol, or a mixture thereof.

Preferably, the alkylphosphine is triphenylphosphine or trioctylphosphine, or a mixture thereof.

The solvent has a boiling point of 100°C or above, and a molecular weight of 100 to 400. In addition, the solvent is selected from the group consisting of hexadecane, hexadecene, octadecane, octadecene, eicosane, eicosene, phenanthrene, pentacene, anthracene, biphenyl, phenyl ether, octyl ether, decyl ether, benzyl ether or squalene, or a mixture thereof.

Preferably, the heating is carried out at a temperature range of 100°C to a boiling point of the solvent, and the rate of the heating is 0.5 °C/min to 50 °C/min. In addition, the pressure of the heating is preferably 0.5 atm to 10 atm.

Preferably, the mole ratio of the metal precursor and the surfactant is 1:0.1 to 1:20 and the mole ratio of the metal precursor and the solvent is 1:1 to 1:1,000.

In the method for preparing the ferrite nanoparticles according to the present invention, when the time period of the heating procedure is significantly short, octahedral ferrite nanoparticles are prepared in large quntities. In addition, when the time period of the heating procedure is slightly short, truncated-cubic ferrite nanoparticles are prepared in large quntities. In contrast, when the time period of the heating procedure is too long, the surfaces of the nanoparticles prepared become rough and coarse.

### Advantageous Effects

It is known that ferrite nanoparticles of a size of 20 nm or above are ferrimagnetic at room temperature. Therefore, when the direction of magnetism of the ferrite nanoparticles of the present invention is used as a unit of information, the information may be stored by controlling the direction of magnetism of the nanoparticles.

In addition, it is possible to access every nanoparticles in cubic arrays in both of the horizontal and vertical directions. In contrast, it is required to movement in a diagonal direction, besides the horizontal and vertical directions, in hexagonal arrays in order to access each nanoparticle. Therefore, it is possible to access the nanoparticles more simply and effectively by the cubic array of the present invention.

### Brief Description of Drawings

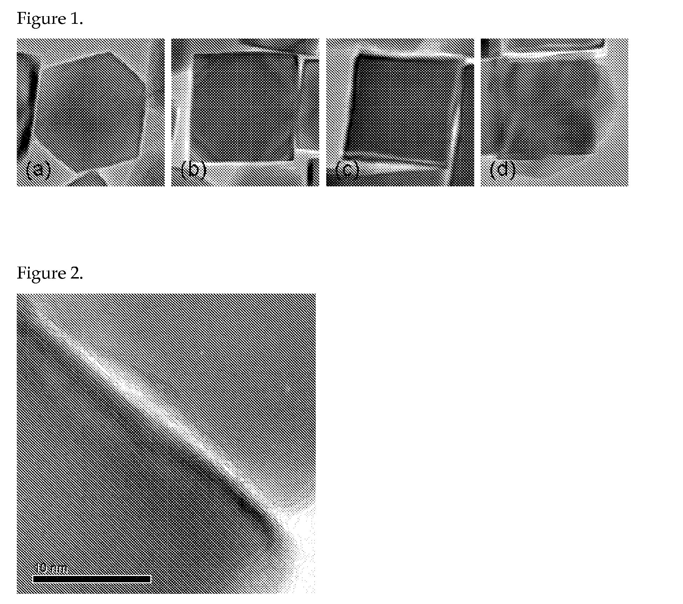

Fig. 1 shows images of the magnetite nanoparticles of various shapes, according to the present invention.
Fig. 2 is a high-resolution transmission electron microscopic image of the magnetite nanocube according to the present invention.

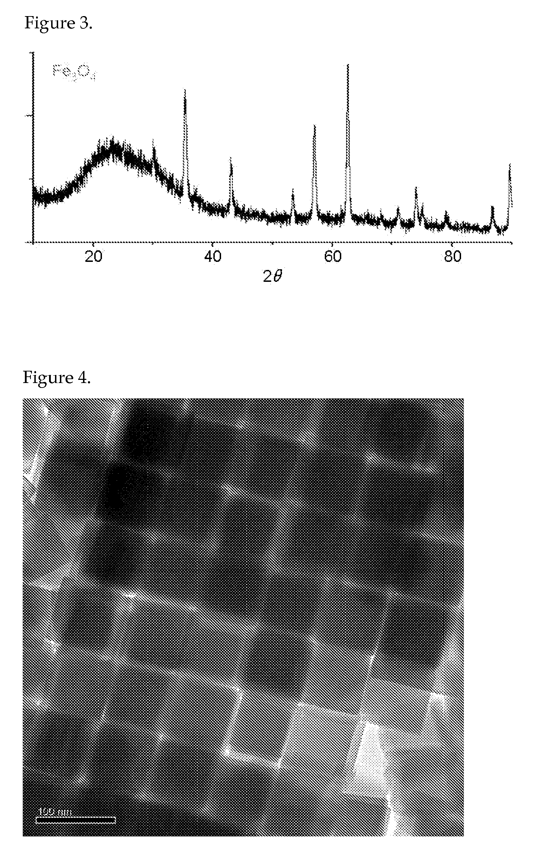

Fig. 3 shows an X-ray diffraction pattern of the magnetite nanocube according to the present invention.
Fig. 4 shows an image of the cubic array of the magnetite nanocube according to the present invention.

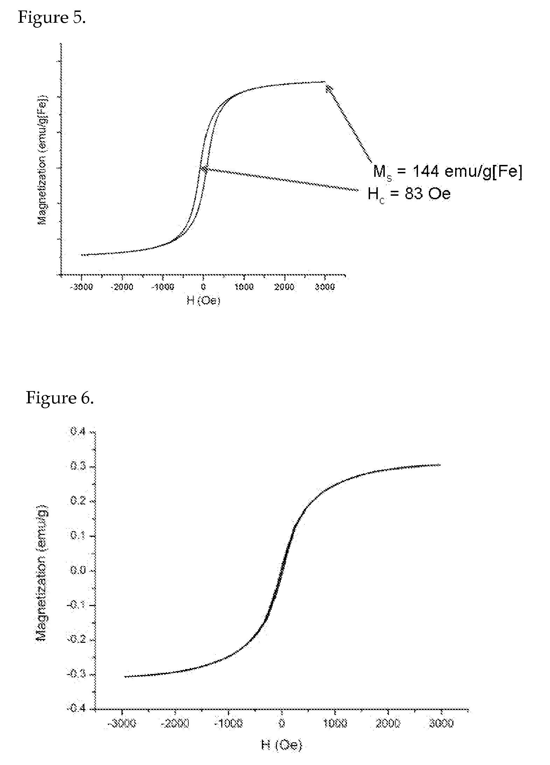

Fig. 5 shows a magnetization curve of the 80 nm-sized magnetite nanocube according to the present invention.
Fig. 6 shows a magnetization curve of the 25 nm-sized magnetite nanocube according to the present invention.

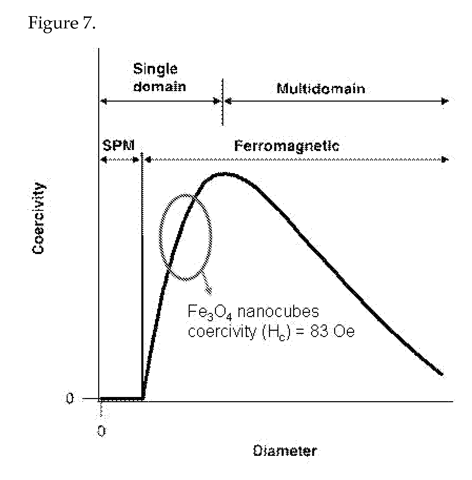

Fig. 7 is a graph indicating that, according to size change of the magnetite nanocubes of the present invention, the magnetism of the nanocubes changes from superparamagnetic to ferrimagnetic, and the structure of the nanocubes changes from single domain to multidomain.

### Best Mode for Carrying Out the Invention

Hereinafter, the present invention will be described in greater detail with reference to the following examples and drawings. The examples and drawings are given only for illustration of the present invention and not to be limiting the present invention.

### Example 1. Preparation of 80 nm-sized magnetite nanocubes

Iron (II) acetylacetonate (0.706 g, 2 mmol) was added to a mixture of oleic acid (1.129 g) and benzyl ether (10.4 g). Remaining air was removed by reducing pressure of the mixture solution via a vacuum pump. Then, the mixture solution was heated up to 290°C with a rate of 20 °C/min, while stirring the mixture solution.

The mixture solution was maintained at 290°C for 30 min. Thereafter, the mixture solution was cooled to 290°C and, then, washed with a mixture of toluene and n-hexane. 80 nm-sized magnetite nanocubes were obtained by centrifuging the washed mixture solution.

### Example 2. Preparation of 100 nm-sized magnetite nanocubes

100 nm-sized magnetite nanocubes were obtained by the same method as Example 1, except for using a rate of 10 °C/min.

### Example 3. Preparation of 20 nm-sized magnetite nanocubes

20 nm-sized magnetite nanocubes were obtained by the same method as Example 1, except for using 20.8 g of benzyl ether and heating time of 2 min.

### Example 4. Preparation of 50 nm-sized magnetite nanocubes

50 nm-sized magnetite nanocubes were obtained by the same method as Example 1, except for using 13 g of benzyl ether and heating time of 15 min.

### Example 5. Preparation of 130 nm-sized magnetite nanocubes

130 nm-sized magnetite nanocubes were obtained by the same method as Example 1, except for using 7.8 g of benzyl ether and heating time of 1 hr.

### Example 6. Preparation of 160 nm-sized magnetite nanocubes

160 nm-sized magnetite nanocubes were obtained by the same method as Example 1, except for using 5.2 g of benzyl ether and heating time of 2 hr.

### Example 7. Shape control of magnetite nanoparticles

About 70 nm-sized truncated-octahedral and truncated-cubic nanoparticles were obtained by the same method as Example 1, except for using 1.271 g of oleic acid. When oleic acid was increased to 1.412 g, the fraction of the truncated-octahedral nanoparticles was increased.

In contrast, about 100 nm-sized rough-surfaced cubic nanoparticles were obtained by the same method as Example 1, except for using 0.989 g of oleic acid. When oleic acid was decreased to 0.847 g, the fraction of the rough-surfaced nanoparticles was increased.

## Claims

1. A ferrite nanocube which is superparamagnetic or ferromagnetic.

2. The ferrite nanocube of Claim 1, wherein the ferrite is Fe₃O₄.

3. The ferrite nanocube of Claim 1, wherein the ferrite is bimetallic.

4. The ferrite nanocube of Claim 3, wherein the bimetallic ferrite is selected from the group consisting of CoFe₂O₄, MnFe₂O₄, ZnFe₂O₄ and BaFe₁₂O₁₉.

5. The ferrite nanocube of Claim 1, wherein the ferrite is Fe₃O₄ doped with a metal.

6. The ferrite nanocube of Claim 5, wherein the metal is selected from the group consisting of Co, Mn, Ni, Zn and Ba.

7. The ferrite nanocube of Claim 1, wherein the ferrite nanocube has a size of 10 nm to 200 nm.

8. A cubic array comprising ferrite nanocubes which are ferrimagnetic.

9. The cubic array of Claim 8, wherein the ferrite is Fe₃O₄.

10. The cubic array of Claim 8, wherein the ferrite is bimetallic.

11. The cubic array of Claim 10, wherein the bimetallic ferrite is selected from the group consisting of CoFe₂O₄, MnFe₂O₄, ZnFe₂O₄ and BaFe₁₂O₁₉.

12. The cubic array of Claim 8, wherein the ferrite is Fe₃O₄ doped with a metal.

13. The cubic array of Claim 12, wherein the metal is selected from the group consisting of Co, Mn, Ni, Zn and Ba.

14. An octahedral or truncated-cubic ferrite nanoparticle which is ferrimagnetic.

15. The ferrite nanoparticle of Claim 14, wherein the ferrite is Fe₃O₄.

16. The ferrite nanoparticle of Claim 14, wherein the ferrite is bimetallic.

17. The ferrite nanoparticle of Claim 16, wherein the bimetallic ferrite is selected from the group consisting of CoFe₂O₄, MnFe₂O₄, ZnFe₂O₄ and BaFe₁₂O₁₉.

18. The ferrite nanoparticle of Claim 14, wherein the ferrite is Fe₃O₄ doped with a metal.

19. The ferrite nanoparticle of Claim 18, wherein the metal is selected from the group consisting of Co, Mn, Ni, Zn and Ba.

20. The ferrite nanoparticle of Claim 18, wherein the ferrite nanoparticle has a size of 10 nm to 200 nm.

21. A method for preparing a ferrite nanocube, comprising heating a mixture of a metal precursor, a surfactant and a solvent.

22. The method of Claim 21, wherein the metal precursor is an iron precursor.

23. The method of Claim 22, wherein the iron precursor is one selected from the group consisting of iron (II) nitrate (Fe(NO₃)₂), iron (III) nitrate (Fe(NO₃)₃), iron (II) sulfate (FeSO₄), iron (III) sulfate (Fe₂(SO₄)₃), iron (II) acetylacetonate (Fe(acac)₂), iron (III) acetylacetonate (Fe(acac)₃), iron (II) trifluoroacetylacetonate (Fe(tfac)₂), iron (III) trifluoroacetylacetonate (Fe(tfac)₃), iron (II) acetate (Fe(ac)₂), iron (III) acetate (Fe(ac)₃), iron (II) chloride (FeCl₂), iron (III) chloride (FeCl₃), iron (II) bromide (FeBr₂), iron (III) bromide (FeBr₃), iron (II) iodide (FeI₂), iron (III) iodide (FeI₃), iron perchlorate (Fe(ClO₄)₃), iron sulfamate (Fe(NH₂SO₃)₂), iron (II) stearate ((CH₃(CH₂)₁₆COO)₂Fe), iron (III) stearate ((CH₃(CH₂)₁₆COO)₃Fe), iron (II) oleate ((CH₃(CH₂)₇CHCH(CH₂)₇COO)₂Fe), iron (III) oleate ((CH₃(CH₂)₇CHCH(CH₂)₇COO)₃Fe), iron (II) laurate ((CH₃(CH₂)₁₀COO)₂Fe), iron (III) laurate ((CH₃(CH₂)₁₀COO)₃Fe), iron pentacarbonyl (Fe(CO)₅), diiron nonacarbonyl (Fe₂(CO)₉) and disodium iron tetracarbonyl (Na₂[Fe(CO)₄]), or a mixture thereof.

24. The method of Claim 21, wherein the metal precursor is a mixture of an iron precursor and one selected from the group consisting of a cobalt precursor, a manganese precursor, a nickel precursor, a zinc precursor and a barium precursor.

25. The method of Claim 24, wherein the cobalt precursor is selected from the group consisting of cobalt (II) nitrate (Co(NO₃)₂), cobalt (II) sulfate (CoSO₄), cobalt (II) acetylacetonate (Co(acac)₂), cobalt (II) trifluoroacetylacetonate (Co(tfac)₂), cobalt (II) acetate (Co(ac)₂), cobalt (II) chloride (CoCl₂), cobalt (II) bromide (CoBr₂), cobalt (II) iodide (CoI₂), cobalt sulfamate (Co(NH₂SO₃)₂), cobalt (II) stearate ((CH₃(CH₂)₁₆COO)₂Co), cobalt (II) oleate ((CH₃(CH₂)₇CHCH(CH₂)₇COO)₂Co), cobalt (II) laurate ((CH₃(CH₂)₁₀COO)₂Co) and dicobalt octacarbonyl (Co₂(CO)₈)).

26. The method of Claim 24, wherein the manganese precursor is selected from the group consisting of manganese (II) nitrate (Mn(NO₃)₂), manganese (II) carbonate (MnCO₃), manganese (III) nitrate (Mn(NO₃)₃), manganese (II) sulfate (MnSO₄), manganese (III) sulfate (Mn₂(SO₄)₃), manganese (II) acetylacetonate (Mn(acac)₂), manganese (III) acetylacetonate (Mn(acac)₃), manganese (II) trifluoroacetylacetonate (Mn(tfac)₂), manganese (III) trifluoroacetylacetonate (Mn(tfac)₃), manganese (II) acetate (Mn(ac)₂), manganese (III) acetate (Mn(ac)₃), manganese (II) chloride (MnCl₂), manganese (II) bromide (MnBr₂), manganese (II) iodide (MnI₂), manganese perchlorate (Mn(ClO₄)₃), manganese sulfamate (Mn(NH₂SO₃)₂), manganese (II) stearate ((CH₃(CH₂)₁₆COO)₂Mn), manganese (III) stearate ((CH₃(CH₂)₁₆COO)₃Mn), manganese (II) oleate ((CH₃(CH₂)₇CHCH(CH₂)₇COO)₂Mn), manganese (III) oleate ((CH₃(CH₂)₇CHCH(CH₂)₇COO)₃Mn), manganese (II) laurate (CH₃(CH₂)₁₀COO)₂Mn), manganese (III) laurate (CH₃(CH₂)₁₀COO)₃Mn), dimanganese decacarbonyl (Mn₂(CO)₁₀) and manganese (II) methoxide (Mn(OMe)₂).

27. The method of Claim 24, wherein the nickel precursor is selected from the group consisting of nickel (II) nitrate (Ni(NO₃)₂), nickel (II) sulfate (NiSO₄), nickel (II) acetylacetonate (Ni(acac)₂), nickel (II) trifluoroacetylacetonate (Ni(tfac)₂), nickel (II) acetate (Ni(ac)₂), nickel (II) chloride (NiCl₂), nickel (II) bromide (NiBr₂), nickel (II) iodide (NiI₂), nickel sulfamate (Ni(NH₂SO₃)₂), nickel (II) stearate ((CH₃(CH₂)₁₆COO)₂Ni), nickel (II) oleate ((CH₃(CH₂)₇CHCH(CH₂)₇COO)₂Ni), nickel (II) laurate ((CH₃(CH₂)₁₀COO)₂Ni) and nickel tetracarbonyl (Ni(CO)₄).

28. The method of Claim 24, wherein the zinc precursor is selected from the group consisting of zinc (II) nitrate (Zn(NO₃)₂), zinc (II) sulfate (ZnSO₄), zinc (II) acetylacetonate (Zn(acac)₂), zinc (II) trifluoroacetylacetonate (Zn(tfac)₂), zinc (II) acetate (Zn(ac)₂), zinc (II) chloride (ZnCl₂), zinc (II) bromide (ZnBr₂), zinc (II) iodide (ZnI₂), zinc sulfamate (Zn(NH₂SO₃)₂), zinc (II) stearate ((CH₃(CH₂)₁₆COO)₂Zn), zinc (II) oleate ((CH₃(CH₂)₇CHCH(CH₂)₇COO)₂Zn), zinc (II) laurate ((CH₃(CH₂)₁₀COO)₂Zn) and zinc (II) tertiary-butoxide (Zn(*t*-butoxide)₂).

29. The method of Claim 24, wherein the barium precursor is selected from the group consisting of barium (II) nitrate (Ba(NO₃)₂), barium (II) sulfate (BaSO₄), barium (II) acetylacetonate (Ba(acac)₂), barium (II) trifluoroacetylacetonate (Ba(tfac)₂), barium (II) acetate (Ba(ac)₂), barium (II) chloride (BaCl₂), barium (II) bromide (BaBr₂), barium (II) iodide (BaI₂), barium sulfamate (Ba(NH₂SO₃)₂), barium (II) stearate ((CH₃(CH₂)₁₆COO)₂Ba), barium (II) oleate ((CH₃(CH₂)₇CHCH(CH₂)₇COO)₂Ba), barium (II) laurate ((CH₃(CH₂)₁₀COO)₂Ba) and barium (II) isopropoxide (Ba(*i-*Pr)₂).

30. The method of Claim 24, wherein the iron precursor is one selected from the group consisting of iron (II) nitrate (Fe(NO₃)₂), iron (III) nitrate (Fe(NO₃)₃), iron (II) sulfate (FeSO₄), iron (III) sulfate (Fe₂(SO₄)₃), iron (II) acetylacetonate (Fe(acac)₂), iron (III) acetylacetonate (Fe(acac)₃), iron (II) trifluoroacetylacetonate (Fe(tfac)₂), iron (III) trifluoroacetylacetonate (Fe(tfac)₃), iron (II) acetate (Fe(ac)₂), iron (III) acetate (Fe(ac)₃), iron (II) chloride (FeCl₂), iron (III) chloride (FeCl₃), iron (II) bromide (FeBr₂), iron (III) bromide (FeBr₃), iron (II) iodide (FeI₂), iron (III) iodide (FeI₃), iron perchlorate (Fe(ClO₄)₃), iron sulfamate (Fe(NH₂SO₃)₂), iron (II) stearate ((CH₃(CH₂)₁₆COO)₂Fe), iron (III) stearate ((CH₃(CH₂)₁₆COO)₃Fe), iron (II) oleate ((CH₃(CH₂)₇CHCH(CH₂)₇COO)₂Fe), iron (III) oleate ((CH₃(CH₂)₇CHCH(CH₂)₇COO)₃Fe), iron (II) laurate ((CH₃(CH₂)₁₀COO)₂Fe), iron (III) laurate ((CH₃(CH₂)₁₀COO)₃Fe), iron pentacarbonyl (Fe(CO)₅), diiron nonacarbonyl (Fe₂(CO)₉) and disodium iron tetracarbonyl (Na₂[Fe(CO)₄]), or a mixture thereof.

31. The method of Claim 21, wherein the surfactant is selected from the group consisting of carboxylic acid, alkylamine, alkyl alcohol and alkylphosphine, or a mixture thereof.

32. The method of Claim 31, wherein the carboxylic acid is selected from the group consisting of octanoic acid, decanoic acid, lauric acid, hexadecanoic acid, oleic acid, stearic acid, benzoic acid and biphenylcarboxylic acid, or a mixture thereof.

33. The method of Claim 31, wherein the alkylamine is selected from the group consisting of octylamine, trioctylamine, decylamine, dodecylamine, tetradecylamine, hexadecylamine, oleylalmine, octadecylamine, tribenzylamine and triphenylamine, or a mixture thereof.

34. The method of Claim 31, wherein the alkyl alcohol is selected from the group consisting of octylalcohol, decanol, hexadecanol, hexadecandiol, oleyl alcohol and phenol, or a mixture thereof.

35. The method of Claim 31, wherein the alkylphosphine is triphenylphosphine or trioctylphosphine, or a mixture thereof.

36. The method of Claim 21, wherein the solvent has a boiling point of 100°C or above, and a molecular weight of 100 to 400.

37. The method of Claim 36, wherein the solvent is selected from the group consisting of hexadecane, hexadecene, octadecane, octadecene, eicosane, eicosene, phenanthrene, pentacene, anthracene, biphenyl, phenyl ether, octyl ether, decyl ether, benzyl ether and squalene, or a mixture thereof.

38. The method of Claim 21, wherein the heating is carried out at a temperature range of 100°C to a boiling point of the solvent.

39. The method of Claim 21, wherein a rate of the heating is 0.5 °C/min to 50 °C /min.

40. The method of Claim 21, wherein a pressure of the heating is 0.5 atm to 10 atm.

41. The method of Claim 21, wherein a mole ratio of the metal precursor and the surfactant is 1:0.1 to 1:20.

42. The method of Claim 21, wherein a mole ratio of the metal precursor and the solvent is 1:1 to 1:1,000.
